# EUROPEAN PATENT SPECIFICATION

(11) **EP 3 079 459 B1**
(45) Date of publication and mention of the grant of the patent: **21.04.2021**
(21) Application number: 14830562.6
(22) Date of filing: 15.12.2014
(51) Int. Cl.: A01G 33/00, A01H 13/00

(54) **CARRIER FOR CULTIVATING ALGAE**
TRÄGER ZUR KULTIVIERUNG VON ALGEN
SUPPORT POUR LA CULTURE D'ALGUES

(30) Priority: 13.12.2013 BE 201300839
(43) Date of publication of application: 19.10.2016
(73) Proprietor: Sioen Industries NV, 8850 Ardooie (BE)
(72) Inventor: GROENENDAAL, Bert, 9910 Knesselare (BE); WILLE, Joost, 8210 Loppem (BE); DE SMET, David, 9900 Eeklo (BE); BUYLE, Guy, 9000 Gent (BE); SCHIPPER, Job, 1705 LN Heerhugowaard (NL); VAN DEN HEUVEL, Freek, 1791 PE Den Burg (Texel) (NL)
(74) Representative: Brantsandpatents bvba
(86) International application number: PCT/IB2014/002785
(87) International publication number: WO 2015/087153

(56) References cited:
- WO-A1-2010/077146
- WO-A1-2012/171123
- WO-A2-2004/093525
- JP-A- H11 262 337
- JP-A- 2001 346 463

## Description

### Technical field

The present invention relates to the cultivation of algae, or algae such as macro-algae and micro-algae, vessels or similar floating structures, adapted for special purposes, and the harvesting of underwater plants, for example, harvesting of algae.

In particular, the invention relates to a carrier for cultivating algae, a method for cultivating algae and the use of a carrier for cultivating algae.

### Background

The cultivating of algae has a long tradition in Asian areas, where it is grown in marine areas according to a labor-intensive process comprising providing seed on ropes, which are then put onto sea and are anchored at multiple points. Hence, these ropes can be positioned horizontally or vertically.

The cultivating of algae is an interesting alternative for obtaining biomass for use in nutrition, food ingredients, animal feed, carrageenan, agar, alginates, and medical applications. A growing interest is recently shown in the use of algae for energy production, such as biogas, bioethanol, electricity, etc., and for pharmaceutical and petrochemical substitutes, as well as for the production of bio-plastics.

The prior art discloses a number of innovative methods to facilitate industrial, automated algae cultivation. It is therein important to increase the yields of algae mass to arrive at an efficient and profitable process.
JP 2003/158928 discloses a bed for the cultivating of algae comprising a flexible plate and a bed onto which spores and the like may attach and grow. A two-dimensional textile structure is thereby described for the cultivation of algae. Preferably, the flexible plate is made of an elastic material with metal particles in order to let the bed sink to the bottom of the sea. The bed can be a textile material from natural or synthetic fiber. Finally, additives to keep away fish are also mentioned.

JP 2002/335784 discloses a bed for the cultivating of macro-algae wherein the seeds or spores are protected against being damaged or eaten by fish. More specifically, the bed is a mat with seeds and thereon a network structure, made from a biodegradable material.

WO 2012/171123 describes compositions, articles, devices, methods, and systems for the growth of algae, immobilized on a carrier in a gaseous environment with the help of sources of carbon dioxide and light, and for subsequent harvesting and processing of biomass. However, such technology requires a very extensive preparation of the textile material, which makes the technique practically very ineffective. WO 2004/093525, in contrast, describes a system for the cultivation of seaweed, such as Enteromorpha clath-rata, in an artificial environment, wherein a shallow container is placed in an aqueous solution for supporting the growth of the algae within the shallow container. Here too, activities are carried out in an artificial environment and the state of the art does not teach how to deal with challenging conditions such as the cultivation algae at sea.

WO 2010/077146 discloses a carrier for growing macro-algae, with an element provided for the growth of seed deposited thereon, and with elements for suspension in a volume of water. The carrier is a plate provided with openings through which water can flow from one side to the other side and wherein the surface of the plate carries the spores and seeds. The invention also comprises a device for the suspension of such a carrier. It consists of connection means connected to at least a portion of an edge of the carrier. It may also be used as a platform submerged in the sea, wherein the carrier can be attached.

JP 2001 346463 discloses a biodegradable net for cultivating laver. JP H11 262337 describes a knotless net for culturing algae.

Despite the above advances in the state of the art, there are still one or more of the following shortcomings:
- known methods provide a relatively low yield of algae which causes the investment costs to be relatively high, which limits the employability;
- the state of the art provides no knowledge or teaching for selecting the most suitable materials for the manufacture of carriers for the cultivation of algae;
- a limited mechanical resistance of materials to currents and/or to marine animals;
- an insufficient adhesion of germs of algae onto the carrier material, whereby the latter will be washed away when going into the sea;
- known structures are inefficient through use of complex carrier structures, which increases the investment costs and requires a labor-intensive process for installation at sea, which ultimately limits employability;
- an undesirable accretion of bio-organisms, such as, for example, micro-organisms or mussels, a phenomenon known as fouling;
- complex, multi-layer systems with carrier structures which are difficult to deploy and do not lend themselves to being re-used;
- low resistance to light, UV and/or aquatic environment and limited lifespan;
- no or only limited opportunities for recycling without a labor-intensive separation process;
- little compact systems, which causes transport cost from production facility to location at sea to entail a considerable cost;
- known structures hardly or do not lend themselves for the mechanical harvesting of seaweed; and
- known structures hardly or do not lend themselves for production on large scale by using prior art industrial plants, making investment costs and cost price not or little attractive for the commercial cultivation of algae.

The current state of the art does not provide or does not adequately provide a solution for one or more of the above-mentioned problems or shortcomings.

### Summary of the invention

To this aim, the invention provides, in a first aspect, a carrier loaded with algae comprising a multi-dimensional textile comprised of a flexible material with a specific surface area greater than 25 m² per m² textile and an average pore size between 25 µm and 500 µm, wherein said textile comprises multifilament yarns.

In a second aspect, the invention provides a method for the cultivation of algae in aquatic environment, comprising the steps of:
- providing a multi-dimensional textile comprised of a flexible material with a specific surface area greater than 25 m² per m² textile and an average pore size between 25 µm and 500 µm in an aquatic environment, wherein said textile comprises multifilament yarns;
- applying algae on said textile, thereby obtaining a carrier, loaded with algae; and
- positioning said carrier by means of fixing and/or floating elements in an aquatic environment.

In a third aspect, the invention provides a use of a carrier according to the first aspect of the invention for the cultivation of algae in aquatic conditions.

In a fourth aspect, the invention provides a kit for cultivating algae, comprising:
(A) carrier according to the first aspect of the invention;
(B) instructions for a user for carrying out a method according to the second aspect of the invention and/or for the use according to the third aspect of the invention.

### Detailed description

Unless defined otherwise, all terms used in the description of the invention, including technical and scientific terms, have the meaning as is commonly understood by the skilled person in the technical field of the invention. For a better assessment of the description of the invention, the following terms are explicitly explained.

"A", "an" and "the" refer in this document to both the singular and the plural unless the context clearly indicates differently. For example, "a segment" means one or more than one segment.

When "about" or "around" is used in this document with a measurable quantity, a parameter, a time period or moment in time, and the like, then variations are implied of +/- 20% or less, preferably +/- 10% or less, more preferably +/- 5% or less, even more preferably +/- 1% or less, and even more preferably +/- 0.1% or less than and of the cited value, to the extent that such variations are applicable in the described invention. It should, however, be understood that the value of the quantity in which the term "about" or "around" is used, is itself specifically disclosed.

The terms "comprise", "comprising', "consist of", "consisting of", "provided with", "contain", "containing", "include", "including", "encompass", "encompassing" are synonyms and are inclusive or open terms that indicate the presence of what follows, and do not exclude or prevent the presence of other components, features, elements, members, steps, known from or described in the prior art.

Quoting of numerical intervals by endpoints includes all integers, fractions and/or real numbers between the endpoints, these endpoints included.

In a first aspect, the invention provides a carrier loaded with algae, comprising a multi-dimensional textile comprised of a flexible material with a specific surface area greater than 25 m² per m² textile and an average pore size between 25 µm and 500 µm, wherein said textile comprises multifilament yarns. This offers the advantage that said textile provides for interstitial spaces in which algae can be fixated.

The term 'multi-dimensional textile' should be understood as a non-one-dimensional textile such as, for example, a line or wire. Preferably, said multi-dimensional textile is a two-dimensional textile such as, for example, a cloth or a three-dimensional textile, such as, for example, a cloth with a well-defined thickness. Most preferably, said multi-dimensional textile has a width, length and thickness.

Preferably, said multi-dimensional textile has a specific surface greater than 25 m², 50 m², 75 m², 100 m², 150 m², 200 m², 250 m², 300 m², 350 m², 400 m² of 450 m² per m² textile or any value in between. In an alternative embodiment, said textile comprises a specific surface area greater than 1000 m² per m² textile.

This offers the advantage that a large specific surface area is provided for the binding of algae onto said carrier. The larger the specific surface area, the more suitable the carrier is for carrying algae. A large specific surface area also provides a high porosity, such that a good diffusion of nutrients from the water to the germs can take place. This promotes the growth of germs into algae.

Preferably, said multi-dimensional textile has an average pore size between 25 µm and 500 µm, most preferably an average pore size of 25 µm, 50 µm, 75 µm, 100 µm, 150 µm, 200 µm, 250 µm, 300 µm, 350 µm, 400 µm, 450 µm or 500 µm, or any value in between.

This offers the advantage that said algae can be fixed in said pores, such that the effective loading of said carrier with said algae is relatively high and, therefore, a high yield can be obtained. Said average pore size can be determined photographically by calculating the average of pore sizes, measured at different positions of said textile.

Preferably, said multi-dimensional textile has an average number of pores per unit volume greater than 100 per cm³, more preferably greater than 1000 per cm³, even more preferably greater than 5000 per cm³, most preferably greater than 10000 per cm³.

One of the advantages thereof is that a sufficient number of pores is present wherein said algae can be fixated. Said number of pores can be photographically determined by calculating the average of counts of pores at different positions of said textile.

Preferably, said multi-dimensional textile comprises a fiber having an average diameter smaller than 1000 µm, more preferably smaller than 500 µm, even more preferably smaller than 200 µm. Most preferably, said fiber diameter ranges between 1 µm and 100 µm, and particularly preferably, said fiber diameter is 5 µm, 10 µm, 15 µm, 20 µm, 25 µm, 30 µm, 40 µm, 50 µm, 60 µm, 70 µm, 80 µm, 90 µm, 100 µm, or any value in between. This offers as advantage that the desired interstitial spaces can be formed wherein algae can be fixated.

The term 'alga' or 'algae' should be understood as a collective term for the terms 'macro-algae' and 'micro-algae', and refers to the members of the family of eukaryotes, ranging from mono-cellular genera such as Chlorella and diatoms to multicellular forms such as large brown algae.

The term 'macro-algae' is to be understood as macro-algae, seaweed, as well as germs, spores, sporophytes, gametophytes and/or seeds of macro-algae which, in aquatic environment, such as saltwater, at sea, and freshwater, grow to become macro-algae under the influence of sunlight. Synonym for the term 'macro-algae' is 'seaweed', 'macro algae', and 'macrophytic sea algae'. Macro-algae comprise a deciduous foliage or thalllus and a root-like attachment organ whereby attachment may be accomplished between macro-algae and a natural carrier such as rocks, coral, other macro-algae, marine animals or an artificial carrier. Preferably, said carrier is an artificial carrier. Said artificial carriers are more readily available and are easier to adjust to productive processes and are not limited by their limited natural occurrence.

The term 'micro-algae' is to be understood as a synonym for the term 'microphytes' or 'microphyte' and refers to the mono-cellular forms such as Chlorella, diatoms and similar adherent species.

In a preferred embodiment, the present invention provides a carrier according to the first aspect of the invention, comprising a textile comprising a material having a contact angle between 40° and 120°, measured after 24 hours of exposure to aquatic environment at a temperature between 10°C and 25°C.

The contact angle is the angle formed between a surface and a drop which is placed on said surface. The size of the contact angle is a measure for the surface tension between said droplet and said surface; the larger the contact angle, the lower the surface tension. Preferably, said contact angle is determined according to ASTM D7334-08 (2013) 'Standard Practice for Surface Wettability of Coatings, Substrates and Pigments by Advancing Contact Angle Measurement'.

Preferably, said contact angle ranges between 50° and 100°, more preferably between 60° and 95°, even more preferably between 70° and 90°. Most preferably, said contact angle is 70°, 75°, 80°, 85° or 90°, or any value in between.

This offers the advantage that an optimum ratio is obtained between, on the one hand, adhesion between germs of algae and textile and, on the other hand, between contact between germs of algae and water. A good adhesion between the germs of algae and textile offers the advantage that the aforementioned does not become detached from said carrier when placed into water or in the sea and then are washed away, which decreases the effective load. A good contact between the germs of algae and water ensures that nutrients from the aquatic environment can be transported to ensure and promote growth.

In a preferred embodiment, the present invention provides a carrier according to the first aspect of the invention, wherein said textile is selected from the group comprising: carpet, non-woven, fabric, knitting and net, or a combination of two or more of said textile configurations.

This offers the advantage that said germs of algae become mechanically attached into the fine microstructure of said textile, improving their adhesion to said textile and yielding a higher efficiency of coverage by germs of algae.

Preferably, said textile is a carpet, a fabric such as, for example, an open fabric or also open structure fabric, or a non-woven, as these have a fine microstructure. Even more preferably, said textile is a non-woven, since a non-woven has a simpler configuration and is therefore easy to produce, to adapt to specific needs and to optimize.

In a preferred embodiment, the present invention provides a carrier according to the first aspect of the invention, said textile comprising a plastic material selected from the group comprising polyethylene, polypropylene, polyester, polylactic acid, polyamide, polyvinyl alcohol, polyvinyl acetate, polyvinyl chloride, polyurethane, polyethylene terephthalate, polyacrylate, polycarbonate, polyalkyl ether, polyvinylidene fluoride, blends and block copolymers of the above; or wherein said textile comprises a natural material such as, for example, but not limited to, cellulose derivatives such as flax, jute, cotton, sisal and bamboo; and wherein said textile is preferably biodegradable and/or biocompatible.

Preferably, said textile comprises a synthetic plastic material. More preferably, said plastic material is selected from the group comprising polypropylene, polyvinyl alcohol, polyamide, polyester and/or blends and/or block copolymers of the aforementioned. Most preferably, said plastic material is selected from the group comprising polyester and polyamide, and/or blends and/or block copolymers of the aforementioned.

This offers the advantage that an optimum stability of said carrier and further higher yields of algae are obtained.

In a preferred embodiment, the present invention provides a carrier according to the first aspect of the invention, wherein said carrier is provided with one or more cut-outs with a diameter of more than 1 cm. More preferably, the cut-outs are provided staggered relative to each other, whereby during use for the cultivation of algae, the incidence of light which is necessary for the growth of the algae, is maximal. Said cut-outs may be provided in a curved or angled form.

Preferably, said cut-out is provided with a diameter of more than 10 cm, more preferably between 25 cm and 100 cm, and most preferably 40 cm, 50 cm, 60 cm or 70 cm, or any value therein between. This is advantageous since algae growth mainly takes place at the edges of said carrier.

In a preferred embodiment, the present invention provides a carrier according to the first aspect of the invention, with a degree of coverage greater than 25% as determined by means of photographic measurement.

More preferably, said degree of coverage ranges between 40% and 100%, even more preferably between 60% and 100%. Most preferably, said degree of coverage is 75%, 80%, 85%, 90%, 95% or 100% or any value therein between.

A high degree of coverage is advantageous as the textile is optimally utilized as carrier loaded with algae. This offers the advantage that said textile is an optimum carrier for germs of algae and that an optimum yield of algae can be provided.

The term 'degree of coverage' refers to the degree of coverage of said textile by germs of algae, and is calculated as the ratio of the surface area of the textile covered by germs of algae relative to the surface area of the textile, expressed in percentage (%).

The degree of coverage is determined by calculating the average of two independent counts of fully-grown germs. The counting is done by visual observation and binocular. The average number of germs per centimeter of textile is determined by taking pictures under a macroscoop of multiple random spots on said textile. The processing of the pictures is done by means of analysis software which is suitable for conducting counts on pictures. The average germ size was determined by taking pictures under a macroscoop of multiple random spots on the textile. Using appropriate analysis software, the length of 30 germs is determined. The average of these measurements is then used to calculate the degree of coverage.

In an alternative embodiment, said degree of coverage is defined as the number of algae which are attached to said carrier per 100 cm² of said carrier, as determined by means of photographic measurement. Preferably, said degree of coverage is greater than 1 macro-algae per 100 cm², more preferably ranges between 1 macro-algae per 100 cm² and 1000 macro-algae per 100 cm², and even more preferably ranges between 10 macro-algae per 100 cm² and 250 macro-algae per 100 cm². Most preferably, said degree of coverage is at 25, 50, 75 or 100 macro-algae per 100 cm² or any value therein between.

Preferably, such a degree of coverage is greater than 1000 micro-algae per 100 cm², more preferably between 1000 micro-algae per 100 cm² and 10 billion micro-algae per 100 cm², and even more preferably between 1 million micro-algae per 100 cm² and 5 billion micro-algae per 100 cm². Most preferably, said degree of coverage is at 1, 2, 3, 4 or 5 billion micro-algae per 100 cm² or any value therein between.

In a preferred embodiment, the present invention provides a carrier according to the first aspect of the invention, wherein said textile comprises a multifilament yarn, and wherein said multifilament yarn comprises spun and/or textured yarns.

This offers the advantage that the above-mentioned filaments ensure a fine structure wherein said germs of algae can be fixated. With a fine structure is meant that said textile has a large specific surface area and a large number of microscopic voids, in particular voids with an average pore size ranging between 1 µm and 2500 µm .

Said mono- and/or multifilament can be spun and can be provided in a twisted or braided configuration. Preferably, said filament is a multifilament, since the adhesion of algae on the textile is much better in the case of multifilament. Still preferably, said multifilament is provided in a twisted configuration, which leads to higher yields.

Preferably, said monofilament or multifilament has a thickness, ranging between 5 dtex and 5000 dtex, more preferably between 10 dtex to 1200 dtex, even more preferably between 15 dtex and 500 dtex. Most preferably, said filament has a thickness of 20 dtex, 40 dtex, 60 dtex, 80 dtex, 100 dtex, 150 dtex, 200 dtex, 250 dtex, 300 dtex, 400 dtex or 500 dtex.

In a preferred embodiment, the present invention provides a carrier according to the first aspect of the invention, wherein said textile is subjected to a physical treatment, such as for example plasma and/or corona treatment. Said plasma and/or corona treatment is mainly advantageous in the case of textile based on polyolefins, such as for example, but not limited to, polyethylene and polypropylene. Said treatments are also suitable to adjust the contact angle between water and textile to an optimum value, more specifically between 40° and 120°, as previously described. Said treatments lead to a better adhesion of algae onto the textile.

In a preferred embodiment, the present invention provides a carrier according to the first aspect of the invention, wherein said textile is provided with a resin for reinforcing said textile.

Each resin known according to the prior art can be advantageously employed in the present invention. For example, both natural and synthetic resins can be used, such as for example, but not limited to, silicone oil. Preferably, said resin is a bio-resin such as for example, but not limited to, bio-resins from cashew nut oil.

This offers the advantage that said carrier obtains a higher structural strength, making it more able to resist mechanical damage or mechanical pressure changes or aging as a result of unwanted contact and/or sea currents.

In an alternative or additional preferred embodiment, the present invention provides a carrier according to the first aspect of the invention, wherein said textile is at least partially provided with a coating for the reinforcement of said textile. Preferably, at least one side of said carrier is coated with a coating for reinforcing said textile. Preferably, said coating comprises polyurethane, polyvinyl chloride, silicone and/or bio-based materials, or a combination of one or more of the above-mentioned materials.

In a preferred embodiment, the present invention provides a carrier according to the first aspect of the invention, wherein said textile is provided with a binding agent for binding algae.

The term 'binding agent' is to be understood as glue, sticking agent or adhesive and is a substance which attaches two or more elements to each other at the level of the respectively contacting surfaces.

This offers the advantage that said binding agent promotes the adhesion of algae to said textile. Said binding agent can be applied separately, in advance onto said textile or can be added to a formulation comprising germs of algae. A suitable binding agent may comprise a polymer, such as for example, but not limited to, methyl cellulose. Preferably, said binding agent is a bio-adhesive.

In a preferred embodiment, the present invention provides a carrier according to the first aspect of the invention, wherein said textile comprises a reinforcement layer.

Said reinforcement layer is preferably comprised out of a net, a knitting, a fabric and/or a non-woven. Also, said reinforcement layer may comprise an edge which provides structural strength to said carrier. This offers as advantage that said carrier obtains a higher structural strength, allowing it to be better able to resist mechanical damage or aging due to unwanted contact and/or sea currents.

Said reinforcement layer may be applied onto said textile or may be incorporated into said textile as a three- or multi-layer textile.

In a preferred embodiment, the present invention provides a carrier according to the first aspect of the invention, wherein said carrier is provided with floating elements for adjusting the average density of said carrier. This offers the advantage that desirable floating properties of said carrier can be obtained in aquatic environment. Said floating elements may be homogeneously or heterogeneously distributed over said carrier.

In a preferred embodiment, the present invention provides a carrier according to the first aspect of the invention, wherein said carrier has a tensile strength greater than 10 N per 5 cm, as measured according to ISO 1421:1998 'Rubber- or plastics-coated fabrics -- Determination of tensile strength and elongation at break'.

Preferably, said carrier has a tensile strength greater than 25 N per 5 cm, more preferably greater than 50 N per 5 cm, even more preferably greater than 100 per 5 cm, most preferably greater than 200 N per 5 cm. It is obvious that higher tensile strengths can also be advantageously used in the present invention.

This offers the advantage that a sufficiently high mechanical strength is obtained, making said carrier more resistant to currents and/or mechanical impact as a result of, for example, floating waste material at sea or organic material such as, for example, branches or trees.

In a preferred embodiment, the present invention provides a carrier according to the first aspect of the invention, wherein said carrier is provided with a layer with nutritional additives.

Preferably, said layer with nutritional additives is applied as a coating onto said textile. Preferably, said nutritional additives comprise phosphorus and/or nitrogen, such as, for example, but not limited to, KH₂PO₄ and NaNO₃.

This offers the advantage that an optimal absorption of said nutritional additives by said germs of algae is obtained.

In a preferred embodiment, the present invention provides a carrier according to the first aspect of the invention, wherein said carrier is provided with attachment means for connecting said carrier with one or multiple fixing and/or floating elements.

Said carrier may (i) be fixed by means of a single point relative to one or multiple fixing elements on the bottom and/or with respect to one or more floating elements which float on the water surface; (ii) be fixed to a horizontal line through a single point; (iii) be fixed to a single vertical line through a single point; (iv) be fixed through multiple points; (v) be fixed laterally to a line; and/or (vi) be fixed laterally to a similar carrier.

This offers the advantage that said carrier can be horizontally and/or be vertically positioned. Preferably, said carrier is fixated just below the water surface for obtaining an optimal absorption of sunlight by said germs of algae and/or by growing algae. This is important since sunlight stimulates the growth of algae through photosynthesis. More preferably, said carrier is positioned at a depth between 0.1 m and 20 m below the water surface, preferably between 0.5 m and 10 m below the water surface, more preferably between 1 m and 5 m below the water surface, most preferably at a depth of 1 m, 2 m, 3 m, 4 m or 5 m below the water surface, or any depth therein between. The stated preferred location for the carrier indicates an optimal depth from below the water surface. Although a greater depth may be associated with a reduced availability of sunlight, it entails the advantage that the carrier is less vulnerable to bad weather conditions.

In a preferred embodiment, the present invention provides a carrier according to the first aspect of the invention, wherein said textile is a non-woven with a density between 10 g/m² and 2000 g/m². Preferably, said non-woven has a density ranging between 100 g/m² and 1000 g/m², more preferably between 150 g/m² and 400 g/m², most preferably equal to 180 g/m², 200 g/m², 230 g/m², 250 g/m², 280 g/m², 300 g/m², 330 g/m², 350 g/m² of 380 g/m² or any value therein between. This offers the advantage that an optimum germ growth and adhesion is obtained. Said density of non-woven offers the advantage that a sufficiently high specific surface area is obtained for the deposition of algae on said textile, and for the creation of the desired interstitial voids for the fixation of said algae.

In a preferred embodiment, the present invention provides a carrier according to the first aspect of the invention, wherein said textile is a non-woven and wherein the filaments of said non-woven are thermally connected to each other by means of a thermal treatment. This offers the advantage that a higher structural strength is obtained.

In a preferred embodiment, the present invention provides a carrier according to the first aspect of the invention, wherein said textile is a non-woven and wherein said non-woven comprises bicomponent yarns, said bicomponent yarn comprising a core comprised of a first plastic and a sheet layer comprised of a second plastic, wherein the melting point of said second plastic is lower than that of said first plastic. When such bicomponent yarn of the non-woven is thermally connected by, for example, heating - and optional pressing together -, this leads to a higher structural strength.

In a preferred embodiment, the present invention provides a carrier according to the first aspect of the invention, wherein said textile is provided on a roll.

In a preferred embodiment, the present invention provides a carrier according to the first aspect of the invention, wherein the carrier is comprised out of a network, wherein the warp and/or weft, and preferably both warp and weft, are comprised out of tape material. In this context, the term 'network' should be broader interpreted as being a mesh structure made comprised of a textile material. The term 'tape material' refers to what, in the professional jargon, is known as 'straps', 'strap' or 'tape' and refers to a material shaped like a tape with a thickness and width, and a virtually unlimited length. Preferably, said network of tape material is provided with a tape width of the tape material ranging between 1 cm and 100 cm, preferably between 5 cm and 50 cm, and more preferably 5 cm, 10 cm, 15 cm, 20 cm, 25 cm, 30 cm, 35 cm or 40 cm, or any width therein between. More preferable, the distance between two consecutive tapes ranges between 1 cm and 100 cm, preferably between 25 cm and 100 cm, and more preferably a distance of 30 cm, 35 cm, 40 cm, 45 cm, 50 cm, 55 cm, 60 cm, 65 cm, 70 cm, 75 cm, 80 cm, 85 cm, 90 cm, or any distance therein between. This offers the advantage that an optimum accessibility of light to the tapes with germ material is obtained. In addition, such a configuration allows the use of less textile material for an equally good or even better yield of seaweed. Thus, higher yields are obtained.

In a preferred embodiment, the present invention provides a carrier according to the first aspect of the invention, wherein two or more of aforementioned preferred embodiments are provided. This offers the advantage that the individual preferred embodiments synergistically complement each other and, consequently, strengthen the intended effects.

In a second aspect, the invention provides a method for the cultivation of algae in aquatic environment, comprising the steps of:
- providing a multi-dimensional textile comprising a flexible material with a specific surface area greater than 25 m² per m² textile and an average pore size between 25 µm and 500 µm in an aquatic environment, and wherein said textile comprises multifilament yarns;
- applying algae on said textile, thereby obtaining a carrier loaded with algae; and
- positioning said carrier by means of fixing and/or floating elements in an aquatic environment.

This provides the advantage that said carrier may be implemented in aquatic environment to obtain a high yield of algae.

The application of algae on said textile can be done both on location and in an industrial environment. The application of algae on location provides the advantage that said textile can immediately be positioned in the aquatic environment. The application of algae in industrial environment helps to ensure a higher quality of application, as well as a homogeneous distribution. Optionally, said textile can be temporarily stored, for example at reduced temperature, but specifically at a temperature between -10°C and 15°C, more preferably between -5 °C and 10 °C.

Preferably, one or more carriers are positioned side by side with an in-between distance greater than 10 cm, more preferably greater than 30 cm and most preferably greater than 60 cm. This offers the advantage that higher yields can be obtained relative to the used surface area of the carrier.

Said germs of algae can be applied to said textile, for transportation to a location at sea or in a location at sea. Optionally, a drying step is used. Preferably, said textile meets one or more of the characteristics of the textile as indicated in the description of the textile of the carrier according to the first aspect of the invention.

In a preferred embodiment, the present invention provides a method according to the second aspect of the invention, wherein, after harvesting of algae, said textile is cleaned, preferably by means of a high-pressure water jet.

This offers the advantage that accreted biomaterial, such as, for example, but not limited to, micro-organisms and mussels, can be separated from said textile without causing damage or significant damage to said textile. Therefore, said textile can be reused for the production of a carrier of algae for cultivating algae. In addition, the use of a carrier material made from one raw material, for example polyamide, offers the advantage that the material after repeated use can also be recycled into new material.

In a third aspect, the invention provides a use of a carrier according to the first aspect of the invention for the cultivation of algae in aquatic environment.

In a fourth aspect, the invention provides a kit for the cultivation of algae, comprising:
(A) carrier according to the first aspect of the invention;
(B) instructions for a user for carrying out a method according to the second aspect of the invention and/or for the use according to the third aspect of the invention.

### Examples

### Example 1

A carrier according to a first embodiment can be obtained by treating a textile with a specific surface area of 45 m² per m² textile and a fiber diameter of 22 µm with a formulation comprising 15 weight-% of young seaweed spores in water and 1 weight-% of sodium methyl cellulose as binding agent. Said carrier may be provided during one harvest season in an aquatic environment and delivers a yield of between 10 kg and 30 kg of algae per m² of textile.

### Example 2

A carrier prepared according to the method described in example 1, wherein said textile is a non-woven which is pre-impregnated with silicone oil, and then heat-treated such that the polyester fibers melt with each other and consequentially a tougher and mechanically more stable carrier is obtained. The non-woven has a tensile strength of 450 N per 5 cm.

### Example 3

Carrier prepared according to the carrier in example 2, wherein on the back of said non-woven, a fabric is provided. The carrier according to this embodiment has a tensile strength of 2850 N per 5 cm.

### Example 4

A textile is sprayed with a diluted suspension of micro-algae until the degree of coverage of said micro-algae on the carrier amounts to 1 billion micro-algae per 100 cm². This carrier is horizontally hung in the sea, for a period of two weeks. Subsequently, the carrier is picked up, and the accrued micro-algae are removed from said carrier with a scraping and suction system.

### Comparative Example 1

A net with a specific surface area of 6 m² per m² net, with a fiber diameter of 750 µm does not provide pores with an average pore size between 1 µm and 2500 µm.

Aforementioned net can be provided in an aquatic environment for one harvest season and provides a yield of less than 1 kg algae per m² textile. This does not meet the expectations and economic needs for obtaining an economically viable yield.

## Claims

1. Carrier loaded with algae, comprising a multidimensional textile comprised of a flexible material with a specific surface area greater than 25 m² per m² textile and an average pore size between 25 µm and 500 µm, wherein said textile comprises multifilament yarns.

2. Carrier according to claim 1, wherein said textile is selected from the group comprising: carpet, non-woven, woven fabric, open structure woven fabric, knitting and net, or a combination of two or more of said textile configurations.

3. Carrier according to claim 1 or 2, wherein said textile comprises a plastic material, selected from the group comprising polyethylene, polypropylene, polyester, polylactic acid, polyamide, polyvinyl alcohol, polyvinyl acetate, polyvinyl chloride, polyurethane, polyethylene terephthalate, polyacrylate, polycarbonate, polyalkyl ether, polyvinylidene fluoride, blends and block copolymers of the above; or wherein said textile comprises a natural material such as, for example, but not limited to, cellulose derivatives such as flax, jute, cotton, sisal and bamboo; and wherein said textile is preferably biodegradable and/or biocompatible.

4. Carrier according to at least one of the preceding claims, with a degree of coverage greater than 1 per 100 cm² macro-algae and/or greater than 1000 micro-algae per 100 cm², as determined by means of photographic measurement.

5. Carrier according to at least one of the preceding claims, wherein said multifilament yarn comprises spun and/or textured yarns.

6. Carrier according to at least one of the preceding claims, wherein said textile comprises one or more twisted multifilament yarns.

7. Carrier according to at least one of the preceding claims, wherein said textile is subjected to a physical treatment, such as, for example, plasma and/or corona treatment.

8. Carrier according to at least one of the preceding claims, wherein said textile is provided with a resin for reinforcing said textile.

9. Carrier according to at least one of the preceding claims, wherein said textile comprises a reinforcement layer.

10. Carrier according to at least one of the preceding claims, wherein said carrier has a tensile strength greater than 10 N per 5 cm, as measured according to ISO 1421:1998.

11. Carrier according to at least one of the preceding claims, wherein said textile is a non-woven, said non-woven comprising bicomponent yarns, said bicomponent yarns comprising a core comprised of a first plastic and a sheet layer of a second plastic, the melting point of said second plastic being lower than that of said first plastic.

12. Method for the cultivation of algae in aquatic environment, comprising the steps of:
- providing a multi-dimensional textile comprised of a flexible material with a specific surface area greater than 25 m² per m² textile and an average pore size between 25 µm and 500 µm, and wherein said textile comprises multifilament yarns;
- applying algae on said textile, thereby obtaining a carrier loaded with algae; and
- positioning said carrier by means of one or multiple fixing and/or floating elements in an aquatic environment.

13. Method according to claim 12, wherein said textile is cleaned after harvesting of algae, preferably by means of a high-pressure water jet.

14. Use of a carrier according to at least one of claims 1 to 11, for cultivating algae in aquatic conditions.

15. Kit for cultivating algae, comprising:
(A) carrier according to at least one of claims 1 to 11;
(B) instructions for a user for carrying out a method according to claim 12 or 13 and/or for the use according to claim 14.

## Patentansprüche

1. Träger, bestückt mit Algen, eine multidimensionale Textilie umfassend, die einem flexiblen Material umfasst mit einer spezifischen Oberfläche von mehr als 25 m² pro m² Textilie und einer durchschnittlichen Porengröße zwischen 25 µm und 500 µm, wobei die Textilie Multifilamentgarne umfasst.

2. Träger nach Anspruch 1, wobei die Textilie aus der Gruppe ausgewählt ist, die umfasst: Teppich, Vlies, Gewebe, Gewebe mit offener Struktur, Gestrick und Netz oder eine Kombination aus zwei oder mehr der Textilgestaltungen.

3. Träger nach Anspruch 1 oder 2, wobei die Textilie ein Kunststoffmaterial umfasst, das aus der Gruppe ausgewählt ist, die umfasst: Polyethylen, Polypropylen, Polyester, Polymilchsäure, Polyamid, Polyvinylalcohol, Polyvinylacetat, Polyvinylchlorid, Polyurethan, Polyethylenterephthalat, Polyacrylat, Polycarbonat, Polyalkylether, Polyvinylidenfluorid, Mischungen und Blockcopolymere des Vorstehenden, oder wobei die Textilie ein natürliches Material umfasst, wie zum Beispiel, aber nicht beschränkt auf, Cellulosederivate, wie beispielsweise Flachs, Jute, Baumwolle, Sisal und Bambus, und wobei die Textilie vorzugsweise biologisch abbaubar und/oder biologisch verträglich ist.

4. Träger nach mindestens einem der vorhergehenden Ansprüche mit einem Bedeckungsgrad von mehr als 1 pro 100 m² Makroalge und/oder mehr als 1000 Mikroalgen pro 100 cm², wie mittels fotografischer Messung bestimmt.

5. Träger nach mindestens einem der vorhergehenden Ansprüche, wobei das Multifilamentgarn gesponnene und/oder texturierte Garne umfasst.

6. Träger nach mindestens einem der vorhergehenden Ansprüche, wobei die Textilie ein oder mehrere verdrillte Multifilamentgarne umfasst.

7. Träger nach mindestens einem der vorhergehenden Ansprüche, wobei die Textilie einer physikalischen Behandlung unterzogen ist, wie zum Beispiel einer Plasma- und/oder einer Coronabehandlung.

8. Träger nach mindestens einem der vorhergehenden Ansprüche, wobei die Textilie mit einem Harz versehen ist, um die Textilie zu verstärken.

9. Träger nach mindestens einem der vorhergehenden Ansprüche, wobei die Textilie einer Verstärkungsschicht umfasst.

10. Träger nach mindestens einem der vorhergehenden Ansprüche, wobei der Träger eine Zugfestigkeit von mehr als 10 N pro 5 cm aufweist, wie gemäß ISO 1421:1998 gemessen.

11. Träger nach mindestens einem der vorhergehenden Ansprüche, wobei die Textilie ein Vlies ist, wobei das Vlies Zweikomponentengarne umfasst, wobei die Zweikomponentengarne einen Kern umfassen, umfassend einem ersten Kunststoff und eine Deckschicht, die einem zweiten Kunststoff umfasst, wobei der Schmelzpunkt des zweiten Kunststoffs niedriger als der des ersten Kunststoffs ist.

12. Verfahren zum Kultivieren von Algen in aquatischer Umgebung, folgende Schritte umfassend:
- Bereitstellen einer multidimensionalen Textilie, umfassend einem flexiblen Material mit einer spezifischen Oberfläche von mehr als 25 m² pro m² Textilie und einer durchschnittlichen Porengröße zwischen 25 µm und 500 µm, wobei die Textilie Multifilamentgarne umfasst,
- Aufbringen von Algen auf die Textilie, wodurch ein mit Algen bestückter Träger erzielt wird, und
- Positionieren des Trägers mittels eines oder mehrerer Fixierungs- und/oder Schwimmelemente in einer aquatischen Umgebung.

13. Verfahren nach Anspruch 12, wobei die Textilie nach dem Ernten der Algen gereinigt wird, vorzugsweise mit einem Hockdruckwasserstrahl.

14. Verwendung eines Trägers nach mindestens einem der Ansprüche 1 bis 11 zum Kultivieren von Algen in aquatischer Umgebung.

15. Kit zum Kultivieren von Algen, umfassend:
(A) Träger nach mindestens einem der Ansprüche 1 bis 11,
(B) Anweisungen für einen Anwender zum Ausführen eines Verfahrens nach Anspruch 12 oder 13 und/oder zur Verwendung nach Anspruch 14.

## Revendications

1. Support chargé d'algues, comprenant un textile multidimensionnel compris d'un matériau souple d'une surface spécifique supérieure à 25 m² par m² de textile et une taille moyenne de pores comprise entre 25 µm et 500 µm, dans lequel ledit textile comprend des fils multifilaments.

2. Support selon la revendication 1, dans lequel ledit textile est choisi dans le groupe comprenant : tapis, non-tissé, tissu tissé, tissu tissé à structure ouverte, tricot et filet, ou une combinaison de deux ou plusieurs desdites configurations textiles.

3. Support selon la revendication 1 ou 2, dans lequel ledit textile comprend une matière plastique, choisie dans le groupe comprenant le polyéthylène, le polypropylène, le polyester, l'acide polylactique, le polyamide, l'alcool polyvinylique, l'acétate de polyvinyle, le chlorure de polyvinyle, le polyuréthane, le polyéthylène téréphtalate, le polyacrylate, le polycarbonate, le polyalkyléther, le polyfluorure de vinylidène, les mélanges et les copolymères séquencés des éléments ci-dessus ; ou dans lequel ledit textile comprend un matériau naturel tel que, par exemple, mais sans s'y limiter, des dérivés de cellulose tels que le lin, le jute, le coton, le sisal et le bambou ; et dans lequel ledit textile est de préférence biodégradable et/ou biocompatible.

4. Support selon au moins l'une des revendications précédentes, avec un degré de couverture supérieur à 1 pour 100 cm² de macro-algues et/ou supérieur à 1000 micro-algues pour 100 cm², déterminé par mesure photographique.

5. Support selon au moins l'une des revendications précédentes, dans lequel ledit fil multifilament comprend des fils filés et/ou texturés.

6. Support selon au moins l'une des revendications précédentes, dans lequel ledit textile comprend un ou plusieurs fils multifilaments torsadés.

7. Support selon au moins l'une des revendications précédentes, dans lequel ledit textile est soumis à un traitement physique, tel que, par exemple, un traitement plasma et/ou corona.

8. Support selon au moins l'une des revendications précédentes, dans lequel ledit textile est pourvu d'une résine pour renforcer ledit textile.

9. Support selon au moins l'une des revendications précédentes, dans lequel ledit textile comprend une couche de renforcement.

10. Support selon au moins l'une des revendications précédentes, dans lequel ledit support a une résistance à la traction supérieure à 10 N par 5 cm, telle que mesurée selon l'ISO 1421:1998.

11. Support selon au moins l'une des revendications précédentes, dans lequel ledit textile est un non-tissé, ledit non-tissé comprenant des fils bicomposés, lesdits fils bicomposés comprenant une âme compris d'un premier plastique et une couche en feuille d'un second plastique, le point de fusion dudit second plastique étant inférieur à celui dudit premier plastique.

12. Procédé de culture d'algues en milieu aquatique, comprenant les étapes de:
- fournir un textile multidimensionnel compris d'un matériau souple avec une surface spécifique supérieure à 25 m² par m² de textile et une taille moyenne de pores comprise entre 25 µm et 500 µm, et dans lequel ledit textile comprend des fils multifilaments;
- appliquer des algues sur ledit textile, obtenant ainsi un support chargé d'algues; et
- positionner ledit support au moyen d'un ou plusieurs éléments de fixation et/ou flottants dans un milieu aquatique.

13. Procédé selon la revendication 12, dans lequel ledit textile est nettoyé après la récolte des algues, de préférence au moyen d'un jet d'eau à haute pression.

14. Utilisation d'un support selon au moins l'une des revendications 1 à 11, pour la culture d'algues en milieu aquatique.

15. Kit de culture d'algues, comprenant :
(A) un support selon au moins l'une des revendications 1 à 11 ;
(B) des instructions destinées à un utilisateur pour la mise en œuvre d'un procédé selon la revendication 12 ou 13 et/ou pour l'utilisation selon la revendication 14.
